# EUROPÄISCHE PATENTANMELDUNG

(11) **EP 4 420 641 A1**
(43) Veröffentlichungstag der Anmeldung: **28.08.2024**
(21) Anmeldenummer: 23000034.1
(22) Anmeldetag: 24.02.2023
(51) Int. Cl.: A61F 6/06

(54) **INFEKTIONSSCHUTZVORRICHTUNG UND KLEIDUNGSSTÜCK**

(71) Anmelder: Sharegh, Atoosa, 81249 München (DE)
(72) Erfinder: Sharegh, Atoosa, 81249 München (DE)

(57) **Zusammenfassung**

Die vorliegende Erfindung betrifft eine Infektionsschutzvorrichtung (100), insbesondere zur Anordnung im Intimbereich einer Oralsex-Empfängerin, wobei die Infektionsschutzvorrichtung (100) umfasst:
- eine Membran (110), wobei die Membran (110) einen Genitalbedeckungsbereich (120) umfasst, und
- einen Positionierungsbereich (102) zur Positionierung der Infektionsschutzvorrichtung (100) am Körper der Oralsex-Empfängerin in einer den Intimbereich ganz oder teilweise überdeckenden Position.

## Beschreibung

Die vorliegende Erfindung betrifft eine Infektionsschutzvorrichtung und ein Kleidungsstück, welches die Infektionsschutzvorrichtung aufweist.

Das US-Patent 3,536,066 beschreibt eine empfängnisverhütende Vorrichtung. Sie umfasst eine einzelne, einstückige, membranartige, elastische Einheit in der Gesamtform und dem Design eines Kleidungsstücks, das einem Höschen im Bikini-Stil sehr ähnlich ist. Dieses Höschen ist mit Vorder- und Rückenteilen versehen, die mit Beinöffnungen durch deren Unterseite verbunden sind, sowie mit einer Öffnung oben zum Eingriff mit dem Oberkörper während des Tragens. Das Höschen hat auch einen Schrittabschnitt mit einem integrierten und umkehrbaren Sackrüssel, der über seine gesamte Länge kreisförmige, balgartige Falten aufweist.

Spätere Veröffentlichungen, die andere Vorrichtungen beschreiben, rücken jeweils nicht die empfängnisverhütende Wirkung, sondern die infektionsverhütende Wirkung beim Oralverkehr in den Vordergrund.

So ist ein Oralschutztuch bekannt. Dieses wird auch als Lecktuch oder Kofferdam bezeichnet. Dabei handelt es sich um eine Folie, die beim Sex auf Vulva oder Anus von Sexualpartnem gelegt wird, um sich beim Oralverkehr vor der Übertragung von krankheitsauslösenden Keimen zu schützen. Das Ansteckungsrisiko für sexuell übertragbare Erkrankung wird durch diese Barriere deutlich vermindert. Das Risiko für die Übertragung von HIV (im Fall von Wunden im Mundraum), HPV, Herpes und Hepatitis sowie von Krankheitserregern aus der Darmflora (z. B. Hepatitis A, Darmparasiten oder Darmbakterien) kann dadurch verringert werden. Als Alternative werden auch aufgeschnittene Kondome, Gummitücher oder reißfeste Haushaltsfolie benutzt.

Das Gebrauchsmuster DE 29607317 U1 beschreibt ein Totalpräservativ für Frauen. Es umfasst einen aus Latexgummi bestehenden Teil, der die äußeren Geschlechtsteile bedeckt und schützt. Es kann darüber hinaus wahlweise einen sich in die Scheide einstülpenden Teil umfassen. Dieser soll vor Eindringen des männlichen Gliedes aufgerollt oder gefaltet sein und sich automatisch beim Eindringen des männlichen Gliedes in die Scheide einstülpen. Das Totalpräservativ weist einvulkanisierte Rundgummis auf, die an einen Hüftgürtel angeknöpft werden und somit dem Präservativ einen sicheren und festen Halt verleihen. Es soll erotisch anregend gestaltet werden können. Ähnlich den Präservativen für Männer soll es in Farbe, Gleitbeschichtung und Geschmack unterschiedlich ausgefertigt werden können. Eine Ausführungsform ohne den sich einstülpenden Teil ist nur zum Schutz bei oralem Geschlechtsverkehr bestimmt.

Das Gebrauchsmuster CN202235794 betrifft ein weibliches Oralsex-Sicherheitshöschen bestehen aus Sicherheitsschutzfolie, Oralsex-Mund, Oralsex-Abdeckung, Sicherheitsschutzfolie-Randstreifen und Sicherheitsschutzfolie-Spitze.

US 2008/302367 A1 beschreibt ein Höschen zum Schutz vor der Übertragung von Infektionskrankheiten beim Oral-, Vaginal- und Analverkehr. Es kann aus Latex oder aus hitzegeprägtem Kunststoff wie Polyurethan hergestellt werden und umfasst zwei Bänder, die sich um die Oberschenkel der Trägerin erstrecken. In dem Dokument wird ebenfalls erwähnt, dass ein Bereich, der die Vagina und den Anus bedeckt, eine breite Palette von Geschmacksrichtungen, eine breite Palette von Größen und eine Vielzahl von Farben, einschließlich transparent, enthalten kann. Es werden zwei Versionen des Höschens beschrieben, eine Oralversion sowie eine Oral- und Penetrationsversion. Die Oral- und Penetrationsversion umfasst zusätzlich einen sackförmigen Bereich für die Penetration. Beide Versionen sollen Gleitmittel auf Wasserbasis (für die Oralversion) und Spermizide (für die Oral- und Penetrationsversion) enthalten.

US 2016/270452 A1 betrifft ein Kleidungsstück zum Durchführen von Oralsex an der Vulva, dem Perineum und/oder Anus. Das Kleidungsstück umfasst: eine aus Elastomermaterial gebildete Membran, wobei die Membran umfasst: einen vorderen Abschnitt; einen hinteren Teil; ein äußerer Oberschenkelabschnitt auf einer rechten Seite der Membran, einen äußeren Oberschenkelabschnitt auf einer linken Seite der Membran; und ein Genitalbereich, wobei der vordere Abschnitt und der hintere Abschnitt der Membran über den äußeren Schenkelabschnitt auf der jeweiligen rechten und linken Seite der Membran verbunden sind, um eine Öffnung an einem oberen Abschnitt der Membran zu bilden, und wobei der vordere Abschnitt und der hintere Abschnitt der Membran über den genitalen Abschnitt verbunden sind und jeder äußere Oberschenkelabschnitt der jeweiligen rechten und linken Seite der Membran über den genitalen Abschnitt verbunden sind, um eine jeweilige Öffnung an jedem des rechten und zu bilden linke Seite der Membran. Als Membran-Material werden beschrieben: Latex, Naturkautschuklatex, synthetischem Latex, Butylkautschuk, Polyethylen, linearem Polyethylen niedriger Dichte (LLDPE), Polyethylen niedriger Dichte (LDPE), Polyethylen hoher Dichte , Polypropylen, Olefin-Copolymer, Styrol-Butadien-Kautschuk (SBR), Polyurethan, Polyisopren, Polyvinylidenchlorid, Polychloropren, carboxylierter Acrylnitril-Butadien-Kautschuk, Nitril, Graphen, Spinifex-Gras, anderes Gras, Nanocellulose, veganes Material, hypoallergenes Material, organisches Material, Superelastomer B, andere Elastomere, andere Polymere, andere Copolymere, andere Polyolefine und eine Kombination beliebiger dieser Materialien. Es wird auch beschrieben, dass die Membran eine verbleibende Substanz von mindestens einem von: einem Schmiermittel, einem Pulver, einem Geschmacksstoff und einem Duftstoff auf mindestens einem Teil der Membran umfassen kann. Die Membran kann außerdem umfassen: Texturwülste im Genitalbereich, eine Ziehharmonikafalte im Genitalbereich, kleine Ausstülpung im Genitalbereich. Es wird auch beschrieben, dass Paare gewöhnliche Plastikfolie, die ursprünglich zum Verpacken und Versiegeln von Lebensmitteln entwickelt wurde, zum Schutz beim Oralsex verwenden.

Der vorliegenden Erfindung liegt die Aufgabe zugrunde, eine Infektionsschutzvorrichtung bereitzustellen, die sowohl für eine Oralsex-Empfängerin als auch für eine mit ihr verkehrende Person ein authentisches Oralsex-Erlebnis ermöglichen und für beide Seiten einen effektiven Schutz bietet, insbesondere gegen eine Übertragung vor HIV, HPV, Herpes und Hepatitis und anderen Krankheiten und Krankheitserreger.

Die erfindungsgemäße Aufgabe wird durch die Merkmale des Anspruchs 1 gelöst. Es wird eine Infektionsschutzvorrichtung bereitgestellt, insbesondere zur Anordnung im Intimbereich einer Oralsex-Empfängerin. Die Infektionsschutzvorrichtung umfasst eine Membran, wobei die Membran einen Genitalbedeckungsbereich umfasst. Die Infektionsschutzvorrichtung umfasst einen Positionierungsbereich zur Positionierung der Infektionsschutzvorrichtung am Körper der Oralsex-Empfängerin in einer den Intimbereich ganz oder teilweise überdeckenden Position.

Die Infektionsschutzvorrichtung ist insbesondere zur Anordnung im Intimbereich einer Oralsex-Empfängerin geeignet. Mit dem Intimbereich der Oralsex-Empfängerin ist in diesem Zusammenhang insbesondere gemeint: Vulva und Perineum (und ggf. Anus), bevorzugt einschließlich umgebender Hautpartien. Als Vulva wird die Gesamtheit der äußeren primären Geschlechtsorgane bestehend aus dem Venushügel, den Schamlippen und der Klitoris bezeichnet.

Die Infektionsschutzvorrichtung umfasst eine Membran. Die Membran besteht im Allgemeinen aus einem für Krankheitserreger im Wesentlichen undurchlässigen Material. Solche Materialien werden z.B. für Kondome oder Oralschutztücher verwendet. Die Membran kann z.B. Latex, Naturkautschuklatex, synthetischen Latex, Butylkautschuk, Polyethylen, lineares Polyethylen niedriger Dichte (LLDPE), Polyethylen niedriger Dichte (LDPE), Polyethylen hoher Dichte (HDPE), Polypropylen, Olefin-Copolymer, Styrol-Butadien-Kautschuk (SBR), Polyurethan, Polyisopren, Polyvinylidenchlorid, Polychloropren, carboxylierter Acrylnitril-Butadien-Kautschuk, Nitril, Graphen, Spinifex-Gras, anderes Gras, Nanocellulose, veganes Material, hypoallergenes Material, organisches Material, Superelastomer B, andere Elastomere, andere Polymere, andere Copolymere, andere Polyolefine und eine Kombination beliebiger dieser Materialien enthalten.

Die Membran kann wenigstens in einem Bereich Verstärkungselemente enthalten. Die Verstärkungselemente können in wenigstens einer Richtung einer Ausdehnung von bis zu 10 µm, bevorzugt bis zu 5 µm, bevorzugt bis zu 1 µm aufweisen. Die Verstärkungselemente können Fasern (z.B. Nanocellulose-Fasem) sein, insbesondere aufweisend einen Faserdurchmesser von bis zu 10 µm, bevorzugt bis zu 5 µm, besonders bevorzugt bis zu 1 µm.

Die Membran umfasst einen Genitalbedeckungsbereich. Der Genitalbedeckungsbereich ist ein Bereich der Membran, der bei bestimmungsgemäßer Verwendung der Infektionsschutzvorrichtung wenigstens die Vulva überdeckt. Die Membran kann einen weiteren Bedeckungsbereich umfassen, z.B. einen Analbedeckungsbereich. Der Analbedeckungsbereich ist ein Bereich der Membran, der bei bestimmungsgemäßer Verwendung der Infektionsschutzvorrichtung den Anus überdeckt. Der Genitalbedeckungsbereich kann ein Bereich der Membran sein, der bei bestimmungsgemäßer Verwendung der Infektionsschutzvorrichtung wenigstens Vulva und Anus überdeckt.

Die Infektionsschutzvorrichtung umfasst einen Positionierungsbereich zur Positionierung der Infektionsschutzvorrichtung am Körper der Oralsex-Empfängerin in einer den Intimbereich ganz oder teilweise überdeckenden Position.

Der Positionierungsbereich kann in die Membran übergehen. Der Positionierungsbereich kann einstückig mit der Membran ausgebildet sein.

Der Positionierungsbereich kann einen Anbindungsbereich umfassen oder ein Anbindungsbereich sein.

Die Infektionsschutzvorrichtung kann über den Anbindungsbereich an einem Kleidungsstück anbindbar oder angebunden sein. Es ist bevorzugt, wenn die Infektionsschutzvorrichtung über den Anbindungsbereich in eine Ausnehmung des Kleidungsstücks einbindbar oder eingebunden ist.

Der Anbindungsbereich kann z.B. zur Anbindung der Infektionsschutzvorrichtung an einen Hautbereich der Oralsex-Empfängerin dienen.

So kann der Positionierungsbereich einen Anbindungsbereich, z. B. den Anbindungsbereich, umfassen oder ein Anbindungsbereich, z. B. der Anbindungsbereich, sein, wobei die Infektionsschutzvorrichtung über den Anbindungsbereich an einem Hautbereich der Oralsex-Empfängerin anbindbar ist.

Der Anbindungsbereich kann z.B. zur Anbindung an einem Kleidungsstück dienen, das eine Ausnehmung zur Aufnahme der Infektionsschutzvorrichtung aufweist.

Der Anbindungsbereich kann auch zur Anbindung der Infektionsschutzvorrichtung an einen Hautbereich der Oralsex-Empfängerin dienen und zur Anbindung an einem solchen Kleidungsstück dienen.

Der Hautbereich kann sich z.B. in den Hautpartien befinden, die Vulva und Perineum (und ggf. Anus) umgeben.

Es kann besonders vorteilhaft sein, wenn die Dicke der Infektionsschutzvorrichtung mindestens in einem Teil des Genitalbedeckungsbereichs geringer ist als die Dicke der Infektionsschutzvorrichtung mindestens in einem Teil des Positionierungsbereichs oder als die Dicke der Infektionsschutzvorrichtung mindestens in einem Teil des Anbindungsbereichs. Dies kann bewirken, dass die Infektionsschutzvorrichtung durch den Genitalbedeckungsbereich eine besonders gefühlsechte Interkation zulässt und diese zugleich mit besonders zuverlässigem Infektionsschutz erfolgen kann. Denn der dickere Anbindungsbereich lässt eine zuverlässigere Anbindung in dem Hautbereich oder an dem Kleidungsstück zu.

Es kann vorteilhaft sein, wenn die Dicke der Infektionsschutzvorrichtung in wenigstens einem Stabilisierungsbereich so groß ist, dass die Infektionsschutzvorrichtung durch den Stabilisierungsbereich in einem entfalteten Zustand gehalten wird. Dies hat den entscheidenden Vorteil, dass die Infektionsschutzvorrichtung auch im erregten Zustand schnell und zuverlässig im Intimbereich der Oralsex-Empfängerin angeordnet werden kann. Ganz anders verhält sich bekanntlich Frischhaltefolie, die sich leicht faltet und an ihren Oberflächen so verklebt, dass sie sich nur noch bei heller Beleuchtung wiederentfalten lässt. Letztlich führt der Stabilisierungsbereich also dazu, dass ein noch höherer Infektionsschutz erreicht werden kann.

Der Stabilisierungsbereich kann ganz oder teilweise mit dem Anbindungsbereich zusammenfallen. Dies hat den Vorteil, dass die Dicke der Infektionsschutzvorrichtung im Wesentlichen im gesamten Genitalbedeckungsbereich sehr dünn gehalten werden kann. Folglich kann eine besonders gefühlsechte Interaktion auf besonders infektionssichere Weise im gesamten Genitalbereich auch bei schlechter Beleuchtung sichergestellt werden.

In Kenntnis der Erfindung ist eine entsprechende Ausführung des Stabilisierungsbereichs für den Fachmann im Rahmen von Routineoptimierungen möglich. Der Stabilisierungsbereich kann z.B. am Rand der Infektionsschutzvorrichtung umlaufend oder an einem Teil des Rands der Infektionsschutzvorrichtung L- oder U-förmig verlaufen, um die Infektionsschutzvorrichtung in dem entfalteten Zustand zu halten.

Im Anbindungsbereich kann ein Anbindungsmittel zur Anbindung der Infektionsschutzvorrichtung angeordnet sein. Denkbar ist aber auch, dass erst unmittelbar vor der Verwendung der Infektionsschutzvorrichtung ein Anbindungsmittel im Anbindungsbereich aufgetragen wird.

Als Anbindungsmittel kommt jedes Mittel in Betracht, mit dem eine Anbindung an dem Hautbereich oder an dem Kleidungsstück möglich ist.

Das Anbindungsmittel kann ein Adhäsionsmittel umfassen. Geeignete Adhäsionsmittel sind dem Fachmann aus Wundpflastem bekannt ist. Es kann bevorzugt sein, wenn das Adhäsionsmittel ein wasserfestes Adhäsionsmittel ist. Dies hat den Vorteil, dass das Adhäsionsmittel sich auch bei einem Schwitzen nur langsam löst, so dass der gewünschte Infektionsschutz ausreichend lange sichergestellt werden kann.

Es kann bevorzugt sein, wenn das Anbindungsmittel ein Klettverschlussanbindungsmittel umfasst.

Das Klettverschlussanbindungsmittel kann z.B. ein Klettverschlussschlaufenelement oder ein Klettverschlusshakenelement sein. Es ist allgemein bekannt, dass Klettverschlüsse Klettverschlusshakenelmente umfassen, die sich beim Schließen des Klettverschlusses in Klettverschlussschlaufenelemente verhaken.

Es kann vorteilhaft sein, wenn der Genitalbedeckungsbereich einen umstülpbaren Bereich umfasst. Unter einem umstülpbaren Bereich wird ein Bereich der Membran verstanden, der sich mit geringem Kraftaufwand, z.B. mit einem Finger oder eine Zunge, von einer der Oralsex-Empfängerin abgewandten Seite auf eine der Oralsex-Empfängerin zugewandte Seite umstülpen lässt. Der umstülpbare Bereich kann z. B. einen rüsselartigen Bereich, insbesondere den aus dem US-Patent Nr. 3,536,066 bekannten Sackrüssel, umfassen.

Die Membran kann in dem umstülpbaren Bereich bevorzugt so dünn uns so weich sein, dass ein Umstülpen des umstülpbaren Bereichs von einer der Oralsex-Empfängerin abgewandten Seite auf eine der Oralsex-Empfängerin zugewandte Seite mit einem Kraftaufwand von weniger als 5 Newton, bevorzugt weniger als 2 Newton, besonders bevorzugt weniger als 1 Newton, z.B. weniger als 0,5 Newton, möglich ist. Dies hat den Vorteil, dass eine mit der Oralsex-Empfängerin interagierende Person fast keinen durch die Infektionsschutzvorrichtung hervorgerufenen Widerstand verspürt, selbst wenn die Interaktion bis in eine Körperöffnung hinein erfolgt. Denn der umstülpbare Bereich kann sich bis in die Körperöffnung hinein erstrecken.

Bevorzugt ist die Infektionsschutzvorrichtung entlang einer Längserstreckungsrichtung der Infektionsschutzvorrichtung gewölbt. Dabei ist die Wölbung entlang der Längserstreckungsrichtung bevorzugt eine Eigenwölbung der Infektionsschutzvorrichtung. Unter einer Eigenwölbung wird in diesem Zusammenhang verstanden, dass die Wölbung durch die Infektionsschutzvorrichtung an sich, z.B. insbesondere durch deren Aufbau und Material, bedingt ist. Mit dem Begriff Eigenwölbung ist also z.B. keine Wölbung einer an sich nicht gewölbten Infektionsschutzvorrichtung gemeint, die erst mit der Anordnung der Infektionsschutzvorrichtung in dem Intimbereich der Oralsex-Empfängerin entsteht.

Dadurch kann der Sitz der Infektionsschutzvorrichtung weiter verbessert werden. Folglich wird das Risiko einer Übertragung der genannten Krankheiten und Krankheitserreger weiter verringert.

Bevorzugt ist die Infektionsschutzvorrichtung entlang einer Quererstreckungsrichtung der Infektionsschutzvorrichtung gewölbt. Dabei ist die Wölbung entlang der Quererstreckungsrichtung bevorzugt eine Eigenwölbung der Infektionsschutzvorrichtung. Unter einer Eigenwölbung wird in diesem Zusammenhang verstanden, dass die Wölbung durch die Infektionsschutzvorrichtung an sich, z.B. insbesondere durch deren Aufbau und Material, bedingt ist. Mit dem Begriff Eigenwölbung ist also z.B. keine Wölbung einer an sich nicht gewölbten Infektionsschutzvorrichtung gemeint, die erst mit der Anordnung der Infektionsschutzvorrichtung in dem Intimbereich der Oralsex-Empfängerin entsteht.

Auch durch die Wölbung entlang der Quererstreckungsrichtung kann der Sitz der Infektionsschutzvorrichtung weiter verbessert werden. Folglich wird auch dadurch das Risiko einer Übertragung der genannten Krankheiten und Krankheitserreger weiter verringert.

Bei der Betrachtung der genannten Wölbungen bleibt ein gegebenenfalls vorliegender umstülpbarer Bereich unberücksichtigt. Es kommt bei der Betrachtung der genannten Wölbungen allein auf den Verlauf der Oberflächen der Infektionsschutzvorrichtung außerhalb des gegebenenfalls vorliegenden umstülpbaren Bereichs an.

Die Längserstreckungsrichtung der Infektionsschutzvorrichtung liegt in einer Ebene, welche sich vertikal entlang der Körperlängsachse der Oralsex-Empfängerin und von vom nach hinten durch die Oralsex-Empfängerin erstreckt, in deren Intimbereich die Infektionsschutzvorrichtung angeordnet ist. Die Quererstreckungsrichtung der Infektionsschutzvorrichtung verläuft orthogonal zur Längserstreckungsrichtung der Infektionsschutzvorrichtung.

Jede der genannten Wölbungen bewirkt, dass die Infektionsschutzvorrichtung einen konkaven Oberflächenbereich aufweist, welcher der Oralsex-Empfängerin zugewandt ist und einen konvexen Oberflächenbereich aufweist, welcher von der Oralsex-Empfängerin abgewandt ist. Durch die beiden Wölbungen entlang der Längserstreckungsachse und der Quererstreckungsachse wird eine Anpassung an korrespondierende Wölbungen der Vulva im Intimbereich der Oralsex-Empfängerin bewirkt, die einerseits den Tragekomfort erhöht und andererseits das Infektionsrisiko beim Oralsex weiter verringert.

Vorteilhaft kann es sein, wenn die Infektionsschutzvorrichtung einen Farbstoff aufweist. Der Farbstoff kann ein Fluoreszenzfarbstoff oder ein Neon-Farbstoff sein, z. B. ein Fluoreszenzfarbstoff.

Vorteilhaft kann es sein, wenn die Infektionsschutzvorrichtung einen Duftstoff und/oder einen Aromastoff aufweist. Es kann sich um einen der Duftstoffe und/oder Aromastoffe handeln, die Fachleuten aus dem Bereich der Kondome bekannt sind. Der Duftstoff und/oder der Aromastoff kann bevorzugt dem Adhäsionsmittel zugesetzt sein.

Vorteilhaft kann es sein, wenn die Infektionsschutzvorrichtung ein Gleitmittel oder ein die Speichelbildung anregendes Mittel aufweist.

Ein weiterer erfindungsgemäßer Gegenstand ist ein Kleidungsstück gemäß Anspruch 11. Das Kleidungsstück weist eine erfindungsgemäße Infektionsschutzvorrichtung auf. Die Infektionsschutzvorrichtung ist über den Anbindungsbereich an dem Kleidungsstück angebunden. Es ist vorteilhaft, wenn die Infektionsschutzvorrichtung über den Anbindungsbereich in die Ausnehmung so in das Kleidungsstück eingebunden ist, dass der Genitalbereich, gegebenenfalls auch der Analbereich, einer Oralsex-Empfängerin, die das Kleidungsstück trägt, durch den Genitalbedeckungsbereich ganz oder teilweise bedeckbar ist.

Das Kleidungsstück kann insbesondere ein Infektionsschutz-Kleidungsstück. Das Infektionsschutz-Kleidungsstück kann insbesondere ein Infektionsschutz-Kleidungsstück zur Anordnung der Membran im Intimbereich der Oralsex-Empfängerin sein.

Das Kleidungsstück kann ein Kleidungsstück in Form einer Hose, z. B. einer Unterhose, sein. Das Kleidungsstück kann z.B. faserbasiert sein, insbesondere naturfaserbasiert oder kunstfaserbasiert. Das Kleidungsstück kann Materialien umfassen, die für Kleidungsstücke und insbesondere Unterwäsche oder Unterhosen üblich sind.

Das Kleidungsstück kann z. B. wenigstens einen textilen Stoff umfassen. Es kann z. B. wenigstens einen Bereich umfassen, der aus einem textilen Stoff gebildet ist.

Das Kleidungsstück kann also z.B. aus einem Material bestehen, das eine Übertragung von Krankheitserregern nicht verhindert. Denn der Genitalbedeckungsbereich der Infektionsschutzvorrichtung kann diejenigen Bereiche zuverlässig bedecken, deren Bedeckung im Zusammenhang mit Krankheitsübertragungen besonders wichtig ist.

Die Ausnehmung kann im Kleidungsstück so angeordnet sein, dass sie sich zumindest um die Vulva oder um die Vulva, das Perineum und den Anus einer Oralsex-Empfängerin erstrecken kann, wenn das Kleidungsstück von der Oralsex-Empfängerin getragen wird.

Das Kleidungsstück kann einen Verstärkungsbereich umfassen, der am Rand der Ausnehmung angeordnet ist. Der Verstärkungsbereich kann die Ausnehmung wenigstens teilweise umgeben.

Das Kleidungsstück kann, z.B. an einem Rand der Ausnehmung, ein Anbindungsmittel umfassen. Das Anbindungsmittel kann ein Klettverschlussanbindungsmittel umfassen.

Das Klettverschlussanbindungsmittel kann z.B. ein Klettverschlussschlaufenelement oder ein Klettverschlusshakenelement sein.

Der Rand der Ausnehmung kann mit dem Anbindungsbereich der Infektionsschutzvorrichtung überlappen, so dass eine Anbindung der Infektionsschutzvorrichtung am Rand der Ausnehmung möglich ist.

Es ist vorteilhaft, wenn das Kleidungsstück ein Verschließelement aufweist, mit dem die Ausnehmung auf einer von dem Intimbereich der Oralsexempfängerin abgewandten Seite der Infektionsschutzvorrichtung so verschließbar ist, dass die Membran durch das Verschließelement ganz oder teilweise überdeckbar ist.

Das Verschließelement kann bevorzugt wenigstens einen Klettverschluss oder einen Reißverschluss, z. B. einen Reißverschluss, umfassen.

Bei einem alternativen erfindungsgemäßen Kleidungsstück, welches eine erfindungsgemäße Infektionsschutzvorrichtung aufweist, erstreckt sich der Positionierungsbereich, optional zusammen mit der Membran, um wenigstens einen Aufnahmebereich herum, in den ein Bein oder ein Bauchbereich der Oralsex-Empfängerin aufnehmbar ist. Es ist bevorzugt, wenn sich der Positionierungsbereich selbst um den wenigstens einen Aufnahmebereich herum erstreckt.

Derartige Aufnahmebereiche weisen Hosen, z. B. Unterhosen auf. Sie weisen typischerweise zwei Aufnahmebereiche auf, in die jeweils ein Bein aufnehmbar ist, und einen Aufnahmebereich auf, in den ein Bauchbereich aufnehmbar ist.

Es ist bevorzugt, wenn sich der Positionierungsbereich selbst um zwei Aufnahmebereiche erstreckt, in die jeweils ein Bein der Oralsex-Empfängerin aufnehmbar ist, und um einen Aufnahmebereich, in den ein Bauchbereich der Oralsex-Empfängerin aufnehmbar ist, erstreckt.

Dieses alternative erfindungsgemäße Kleidungsstück kann zu wenigstens 50 Gew.-% aus Latex, Naturkautschuklatex, synthetischem Latex, Butylkautschuk, Polyethylen, linearem Polyethylen niedriger Dichte (LLDPE), Polyethylen niedriger Dichte (LDPE), Polyethylen hoher Dichte (HDPE), Polypropylen, Olefin-Copolymer, Styrol-Butadien-Kautschuk (SBR), Polyurethan, Polyisopren, Polyvinylidenchlorid, Polychloropren, carboxylierter Acrylnitril-Butadien-Kautschuk, Nitril, Graphen, Spinifex-Gras, anderem Gras, Nanocellulose, veganem Material, hypoallergenem Material, organischem Material, Superelastomer B, anderen Elastomeren, anderen Polymeren, anderen Copolymeren, anderen Polyolefinen und einer Kombination beliebiger dieser Materialien bestehen.

Dieses alternative erfindungsgemäße Kleidungsstück kann einstückig sein. Es kann weniger als 50 Gew.-%, bevorzugt weniger als 20 Gew.-%, an textilem Stoff aufweisen. Es kann z. B. keinen textilen Stoff aufweisen.

Die Erfindung wird durch die nachfolgenden Figuren veranschaulicht, ohne auf die dort gezeigten Ausführungsformen beschränkt zu sein.
Figuren 1 bis 4 zeigen eine erste Ausführungsform einer Infektionsschutzvorrichtung.
Figuren 5 bis 8 zeigen eine zweite Ausführungsform einer Infektionsschutzvorrichtung.

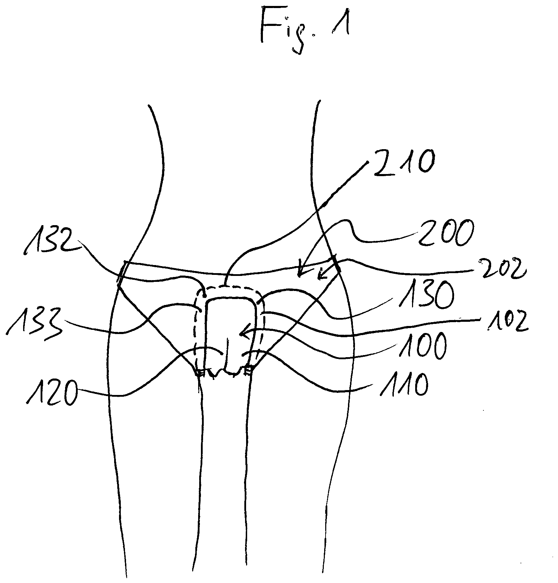

Die in Figur 1 gezeigte erste Ausführungsform einer Infektionsschutzvorrichtung 100 umfasst eine Membran 110 und einen Anbindungsbereich 130. Die Membran umfasst einen Genitalbedeckungsbereich 120.

Im Anbindungsbereich 130 ist ein Anbindungsmittel 132 zur Anbindung der Infektionsschutzvorrichtung 100 angeordnet. Das Anbindungsmittel 132 ist ein Klettverschlussanbindungsmittel 133.

Figur 1 zeigt auch ein Kleidungsstück 200 in Form einer Unterhose, wobei das Kleidungsstück 200 eine Ausnehmung 210 zur Aufnahme der Infektionsschutzvorrichtung 100 aufweist. Über das Klettverschlussanbindungsmittel 133 ist der Anbindungsbereich 130 an einem Rand der Ausnehmung 210 angebunden.

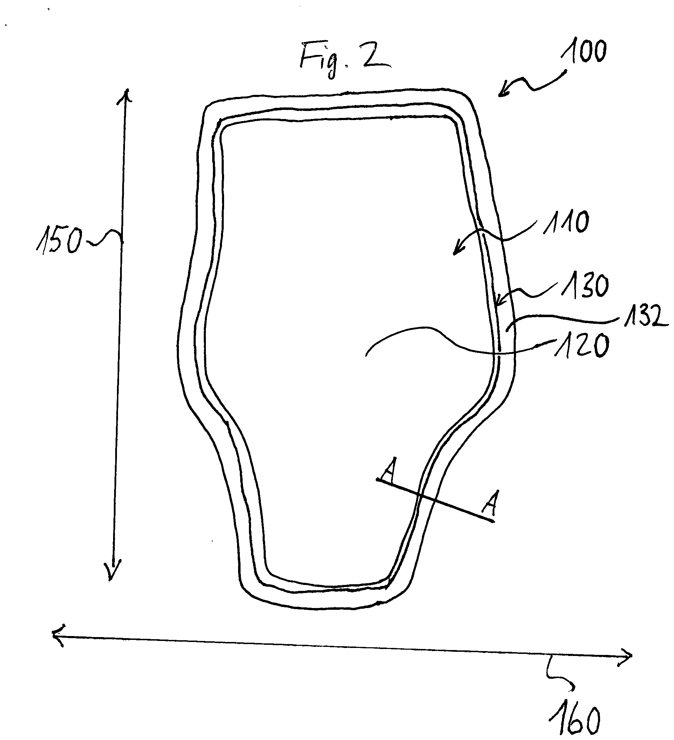

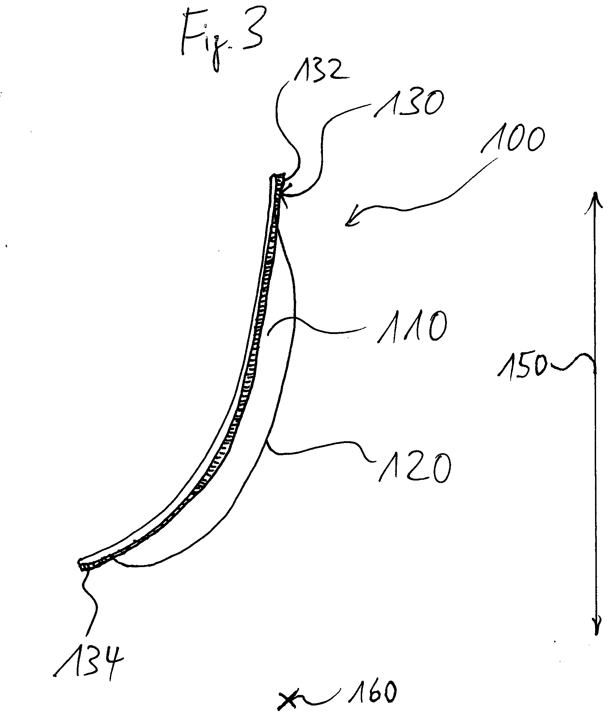

Figur 2 und 3 zeigen jeweils die Infektionsschutzvorrichtung 100 aus Figur 1.

In Figur 2 ist dem Betrachter eine Oberfläche der Infektionsschutzvorrichtung zugewandt. Diese Oberfläche ist von der Oralsex-Empfängerin, die in Figur 1 angedeutet ist, abgewandt. Das Anbindungsmittel 132 am Anbindungsbereich 130 ist auf einer von der Oralsex-Empfängerin abgewandten Oberfläche angeordnet. Das Anbindungsmittel 132 ist zur Membran 110 beabstandet.

In Figur 2 sind auch eine Längserstreckungsrichtung 150 und eine Quererstreckungsrichtung 160 eingezeichnet.

In Figur 3 ist die Infektionsschutzvorrichtung 100 von der Seite dargestellt. Die Blickrichtung des Betrachters verläuft entlang der Quererstreckungsrichtung 160, die dort deshalb nur mit einem Kreuz angedeutet ist. Figur 3 zeigt noch deutlicher, dass das Anbindungsmittel 132 am Anbindungsbereich 130 auf einer von der Oralsex-Empfängerin abgewandten Oberfläche angeordnet ist. Das Anbindungsmittel 132 umfasst Klettverschlussschlaufenelemente 134.

Die Infektionsschutzvorrichtung 100 ist entlang der Längserstreckungsrichtung 150 und entlang der Quererstreckungsrichtung 160 gewölbt. Dies ist in Figur 3 deutlich zu erkennen.

Der Genitalbedeckungsbereich 120 verläuft in Figur 3 deshalb rechts versetzt zum Anbindungsbereich 130.

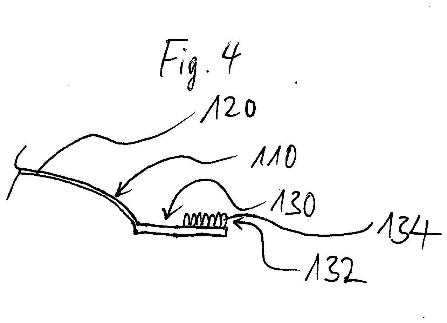

Die Wölbung entlang der Quererstreckungsrichtung 160 ist auch in Figur 4 gut zu erkennen. Dort ist der Schnitt A-A aus Figur 2 dargestellt. Aufgrund der Wölbung entlang der Quererstreckungsrichtung 160 verläuft in Figur 4 der Genitalbedeckungsbereich 120 oberhalb des Anbindungsbereichs 130.

Die Wölbung ist eine Eigenwölbung. Figur 4 zeigt auch, dass die Dicke der Infektionsschutzvorrichtung im gezeigten Teil des Genitalbedeckungsbereichs 120 geringer ist als die Dicke der Infektionsschutzvorrichtung im gezeigten Teil des Anbindungsbereichs 130.

Der Anbindungsbereich 130 kann zugleich als Stabilisierungsbereich aufgefasst werden. Dort ist die Dicke so groß ist, dass die Infektionsschutzvorrichtung in einem entfalteten Zustand gehalten wird, der zugleich die Eigenwölbung entlang der beiden Erstreckungsrichtungen 150 und 160 aufrechterhält. Der Stabilisierungsbereich fällt in dem gezeigten Beispiel also mit dem Anbindungsbereich 130 zusammenfallen.

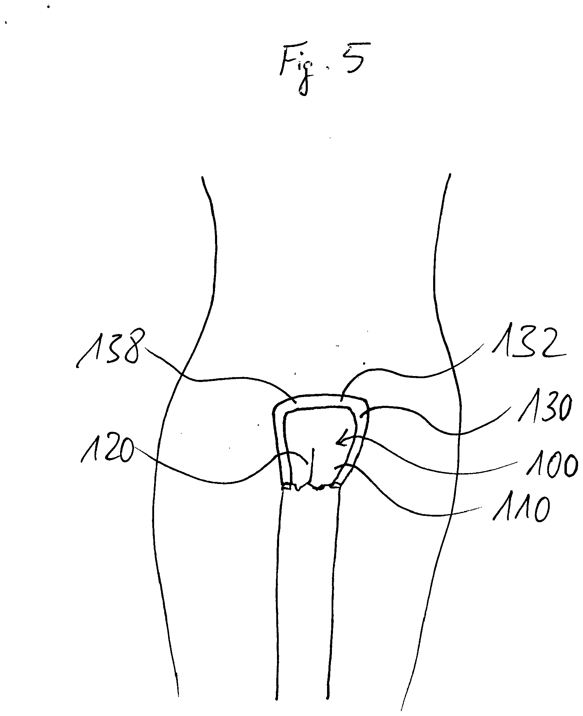

Die in Figur 5 gezeigte zweite Ausführungsform einer Infektionsschutzvorrichtung 100 umfasst eine Membran 110 und einen Anbindungsbereich 130. Die Membran umfasst einen Genitalbedeckungsbereich 120. Als Anbindungsmittel 132 dient ein Adhäsionsmittel 138.

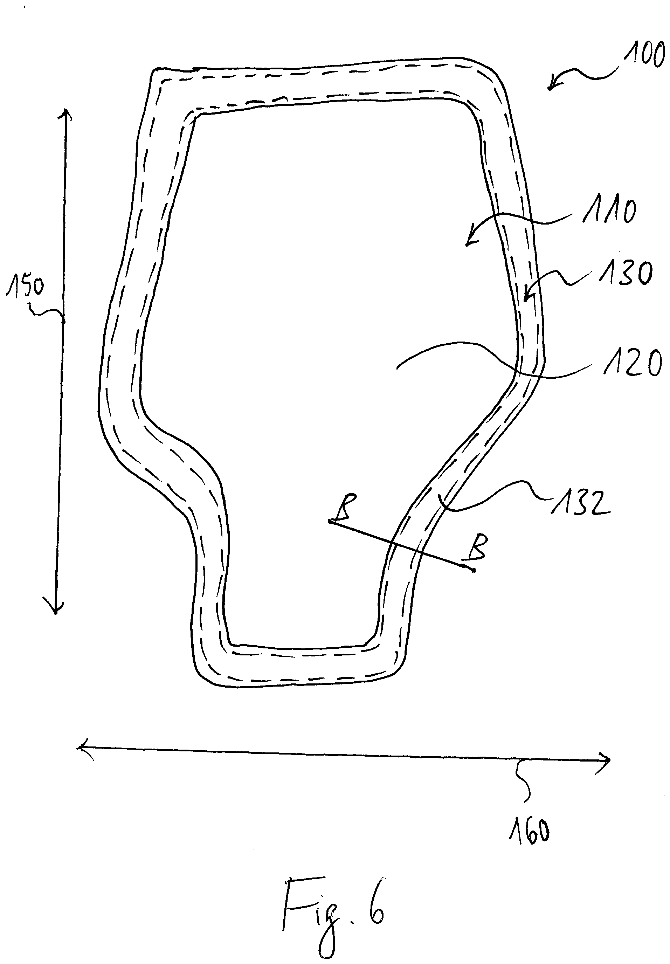

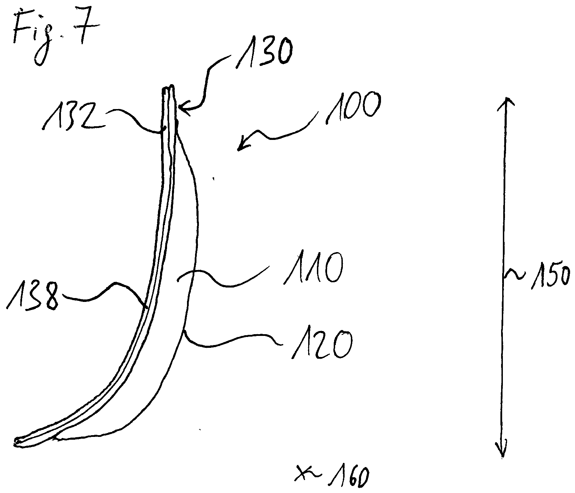

Figur 6 und 7 zeigen jeweils die Infektionsschutzvorrichtung 100 aus Figur 5.

In Figur 6 ist dem Betrachter eine Oberfläche der Infektionsschutzvorrichtung zugewandt. Diese Oberfläche ist von der Oralsex-Empfängerin, die in Figur 5 angedeutet ist, abgewandt. Das Anbindungsmittel 132 ist am Anbindungsbereich 130 auf einer der Oralsex-Empfängerin zugewandten Oberfläche angeordnet und der Bereich, in dem sich das Anbindungsmittel befindet, daher durch zwei gestrichelte Linien angedeutet.

In Figur 6 sind auch eine Längserstreckungsrichtung 150 und eine Quererstreckungsrichtung 160 eingezeichnet.

In Figur 7 ist die Infektionsschutzvorrichtung 100 von der Seite dargestellt. Die Blickrichtung des Betrachters verläuft entlang der Quererstreckungsrichtung 160, die dort deshalb nur mit einem Kreuz angedeutet ist. Figur 7 zeigt noch deutlicher, dass das Anbindungsmittel 132, d.h. das Adhäsionsmittel 138, am Anbindungsbereich 130 auf einer der Oralsex-Empfängerin zugewandten Oberfläche angeordnet ist.

Die Infektionsschutzvorrichtung 100 ist entlang der Längserstreckungsrichtung 150 und entlang der Quererstreckungsrichtung 160 gewölbt. Dies ist in Figur 7 deutlich zu erkennen. Der Genitalbedeckungsbereich 120 verläuft in Figur 7 deshalb rechts versetzt zum Anbindungsbereich 130.

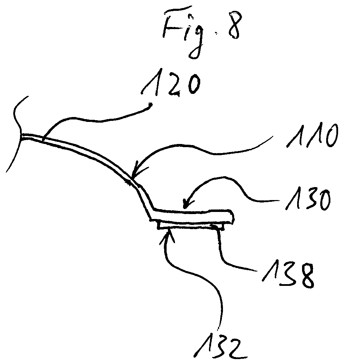

Die Wölbung entlang der Quererstreckungsrichtung 160 ist auch in Figur 8 gut zu erkennen. Dort ist der Schnitt B-B aus Figur 6 dargestellt. Aufgrund der Wölbung entlang der Quererstreckungsrichtung 160 verläuft in Figur 8 der Genitalbedeckungsbereich 120 oberhalb des Anbindungsbereichs 130.

Die Wölbung ist eine Eigenwölbung. Figur 8 zeigt auch, dass die Dicke der Infektionsschutzvorrichtung im gezeigten Teil des Genitalbedeckungsbereichs 120 geringer ist als die Dicke der Infektionsschutzvorrichtung im gezeigten Teil des Anbindungsbereichs 130.

Der Anbindungsbereich 130 kann zugleich als Stabilisierungsbereich aufgefasst werden. Dort ist die Dicke so groß ist, dass die Infektionsschutzvorrichtung in einem entfalteten Zustand gehalten wird, der zugleich die Eigenwölbung entlang der beiden Erstreckungsrichtungen 150 und 160 aufrechterhält. Der Stabilisierungsbereich fällt in dem gezeigten Beispiel also mit dem Anbindungsbereich 130 zusammenfallen.

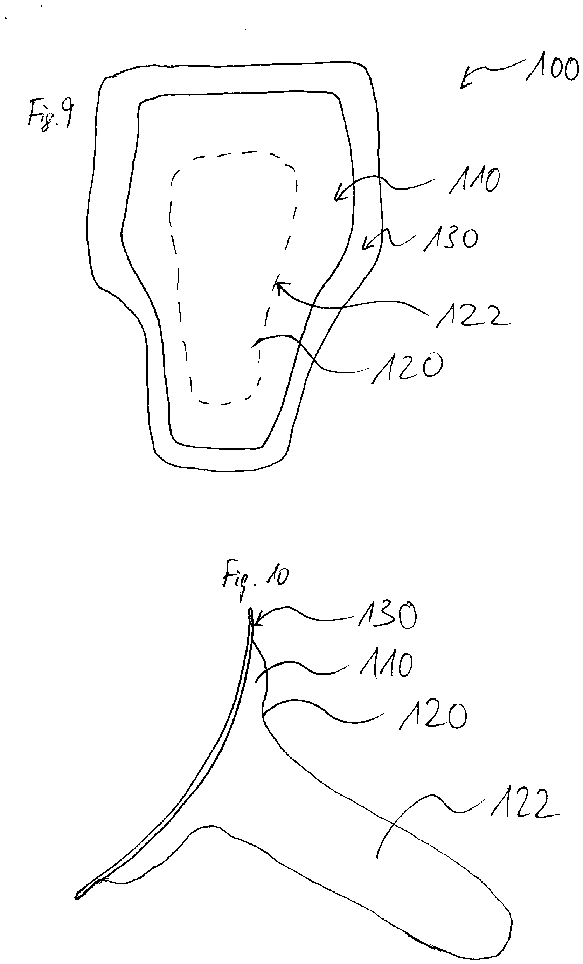

Figuren 9 und 10 veranschaulichen eine Weiterbildungsmöglichkeit der beiden in Figuren 1 bis 4 und in Figuren 5 bis 8 gezeigten Ausführungsformen. Der in Figuren 9 und 10 gezeigte Genitalbedeckungsbereich 120 umfasst einen umstülpbaren Bereich 122 in Form eines Sackrüssels, der hier in ausgestreckter Form dargestellt ist.

Figur 9 entspricht den Ansichten in Figuren 2 und 6. Zusätzlich ist in Figur 9 der umstülpbare Bereich 122 gestrichelt angedeutet.

Die Ansicht in Figur 10 entspricht den Ansichten in Figuren 3 und 7. In der gezeigten Ansicht ist der umstülpbare Bereich nur zu Illustrationszwecken auf eine von der Oralsex-Empfängerin abgewandte Seite der Infektionsschutzvorrichtung umgestülpt. Selbstverständlich kann der umstülpbare Bereich 122 mit äußerst geringem Kraftaufwand auf die andere Seite umgestülpt werden, und so zwischen die Schamlippen der Oralsex-Empfängerin bewegt werden. Die gezeigte Ausführung des umstülpbaren Bereichs reicht zur Aufnahme eines männlichen Glieds aus. Für den Oralverkehr reicht es selbstverständlich aus, wenn ein umgestülpter Abschnitt des umgestülpten Bereichs deutlich kürzer ist.

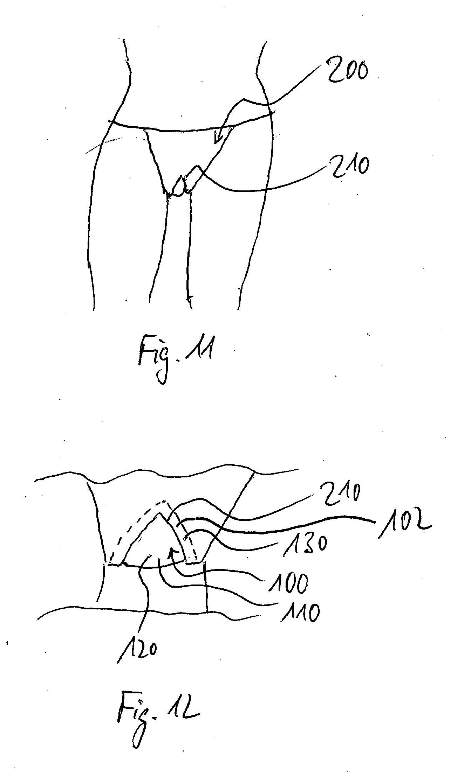

Figuren 11 bis 14 zeigen erfindungsgemäße Kleidungsstücke 200 in Form von Unterhosen, die Infektionsschutz-Kleidungsstücke 202 darstellen. Figuren 12 bis 14 zeigen jeweils nur einen vergrößerten Ausschnitt.

Die in Figuren 11 bis 14 gezeigten Kleidungsstücks 200 umfassen je eine Infektionsschutzvorrichtung 100 und eine Ausnehmung 210. Die Infektionsschutzvorrichtung 100 ist jeweils über den Anbindungsbereich 130 an dem Kleidungsstück 200 angebunden. Die Infektionsschutzvorrichtung 100 ist über den Anbindungsbereich 130 in die Ausnehmung 210 jeweils so in das Kleidungsstück eingebunden, dass der Genitalbereich einer Oralsex-Empfängerin, die das Kleidungsstück 200 trägt, durch den Genitalbedeckungsbereich 120 ganz bedeckt ist.

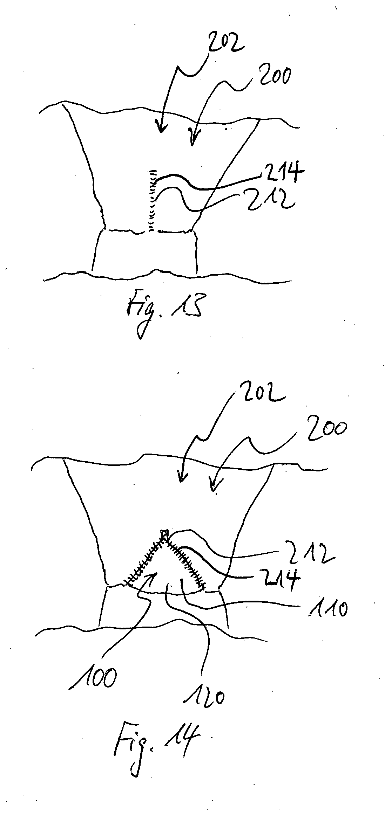

Das in Figuren 13 und 14 gezeigte Kleidungsstück weist ein Verschließelement 212 auf, das einen Reißverschluss 214 umfasst. Mit dem Verschließelement 212 ist die Ausnehmung 210 auf einer von dem Intimbereich der Oralsexempfängerin abgewandten Seite der Infektionsschutzvorrichtung so verschließbar, dass die Membran 110 durch das Verschließelement 212 überdeckbar ist. Figur 13 zeigt den verschlossenen Zustand, wohingegen Figur 14 den geöffneten Zustand zeigt.

**Bezugszeichenliste**

| | |
|---|---|
| **Infektionsschutzvorrichtung** | **100** |
| **Positionierungsbereich** | **102** |
| **Membran** | **110** |
| **Genitalbedeckungsbereich** | **120** |
| **umstülpbarer Bereich** | **122** |
| **Anbindungsbereich** | **130** |
| **Anbindungsmittel** | **132** |
| **Klettverschlussanbindungsmittel** | **133** |
| **Klettverschlussschlaufenelement** | **134** |
| **Klettverschlusshakenelement** | **135** |
| **Adhäsionsmittel** | **138** |
| **Hautbereich** | **140** |
| **Längserstreckungsrichtung** | **150** |
| **Quererstreckungsrichtung** | **160** |
| **Kleidungsstück** | **200** |
| **Infektionsschutz-Kleidungsstück** | **202** |
| **Ausnehmung** | **210** |
| **Verschließelement** | **212** |
| **Reißverschluss** | **214** |

## Patentansprüche

1. Infektionsschutzvorrichtung (100), insbesondere zur Anordnung im Intimbereich einer Oralsex-Empfängerin, wobei die Infektionsschutzvorrichtung (100) umfasst:
- eine Membran (110), wobei die Membran (110) einen Genitalbedeckungsbereich (120) umfasst, und
- einen Positionierungsbereich (102) zur Positionierung der Infektionsschutzvorrichtung (100) am Körper der Oralsex-Empfängerin in einer den Intimbereich ganz oder teilweise überdeckenden Position.

2. Infektionsschutzvorrichtung (100) nach Anspruch 1, wobei der Positionierungsbereich (102) einen Anbindungsbereich (130) umfasst oder ein Anbindungsbereich (130) ist, wobei die Infektionsschutzvorrichtung (100) über den Anbindungsbereich (130) an einem Kleidungsstück (200) anbindbar oder angebunden ist, wobei es bevorzugt ist, wenn die Infektionsschutzvorrichtung (100) über den Anbindungsbereich (130) in eine Ausnehmung (210) des Kleidungsstücks (200) einbindbar oder eingebunden ist.

3. Infektionsschutzvorrichtung (100) nach Anspruch 1 oder 2, wobei die Dicke der Infektionsschutzvorrichtung (100) mindestens in einem Teil des Genitalbedeckungsbereichs (120) geringer ist als die Dicke der Infektionsschutzvorrichtung (100) mindestens in einem Teil des Positionierungsbereichs (102) oder mindestens in einem Teil des Anbindungsbereichs (130).

4. Infektionsschutzvorrichtung (100) nach Anspruch 2 oder 3, wobei im Anbindungsbereich (130) ein Anbindungsmittel (132) zur Anbindung der Infektionsschutzvorrichtung (100) angeordnet ist.

5. Infektionsschutzvorrichtung (100) nach Anspruch 4, wobei das Anbindungsmittel (132) ein Klettverschlussanbindungsmittel (133), z.B. ein Klettverschlussschlaufenelement (134) oder ein Klettverschlusshakenelement (135), umfasst.

6. Infektionsschutzvorrichtung (100) nach Anspruch 4, wobei das Anbindungsmittel (132) ein Adhäsionsmittel (138) umfasst.

7. Infektionsschutzvorrichtung (100) nach einem der vorhergehenden Ansprüche, wobei der Genitalbedeckungsbereich (120) einen umstülpbaren Bereich (122) umfasst.

8. Infektionsschutzvorrichtung (100) nach einem der vorhergehenden Ansprüche, wobei die Infektionsschutzvorrichtung (100) entlang einer Längserstreckungsrichtung (150) der Infektionsschutzvorrichtung gewölbt ist und/oder entlang einer Quererstreckungsrichtung (160) der Infektionsschutzvorrichtung gewölbt ist.

9. Infektionsschutzvorrichtung (100) nach einem der vorhergehenden Ansprüche, wobei die Infektionsschutzvorrichtung (100) einen Farbstoff aufweist, bevorzugt einen Fluoreszenzfarbstoff.

10. Infektionsschutzvorrichtung (100) nach einem der vorhergehenden Ansprüche, wobei die Infektionsschutzvorrichtung (100) einen Duftstoff und/oder einen Aromastoff aufweist, wobei der Duftstoff und/oder der Aromastoff bevorzugt dem Adhäsionsmittel zugesetzt sein kann.

11. Kleidungsstück (200), wobei das Kleidungsstück (200) eine Infektionsschutzvorrichtung (100) nach einem der Ansprüche 2 bis 10 und eine Ausnehmung (210) aufweist,
wobei die Infektionsschutzvorrichtung (100) über den Anbindungsbereich (130) an dem Kleidungsstück (200) angebunden ist und die Infektionsschutzvorrichtung (100) über den Anbindungsbereich (130) in die Ausnehmung (210) so in das Kleidungsstück eingebunden ist, dass der Genitalbereich einer Oralsex-Empfängerin, die das Kleidungsstück (200) trägt, durch den Genitalbedeckungsbereich (120) ganz oder teilweise bedeckbar ist.

12. Kleidungsstück (200) nach Anspruch 11,
wobei das Kleidungsstück ein Verschließelement (212) aufweist, mit dem die Ausnehmung (210) auf einer von dem Intimbereich der Oralsexempfängerin abgewandten Seite der Infektionsschutzvorrichtung so verschließbar ist, dass die Membran (110) durch das Verschließelement (212) ganz oder teilweise überdeckbar ist.

13. Kleidungsstück (200) nach Anspruch 12,
wobei das Verschließelement wenigstens einen Klettverschluss oder einen Reißverschluss (214) umfasst.

14. Kleidungsstück (200), wobei das Kleidungsstück (200) die Infektionsschutzvorrichtung (100) nach einem der Ansprüche 1, 3 oder 7 bis 10 aufweist,
wobei der Positionierungsbereich (210) sich, optional zusammen mit der Membran (110), um wenigstens einen Aufnahmebereich herum erstreckt, in den ein Bein oder ein Bauchbereich der Oralsex-Empfängerin aufnehmbar ist.

15. Infektionsschutzvorrichtung (100) nach einem der Ansprüche 1, 3, 4 oder 6 bis 10, wobei der Positionierungsbereich (102) einen Anbindungsbereich (130), z. B. den Anbindungsbereich (130), umfasst oder ein Anbindungsbereich (130), z. B. der Anbindungsbereich (130), ist, wobei die Infektionsschutzvorrichtung (100) über den Anbindungsbereich (130) an einem Hautbereich (140) der Oralsex-Empfängerin anbindbar ist.
